# EUROPÄISCHE PATENTSCHRIFT

(11) **EP 0 699 753 B1**
(45) Veröffentlichungstag und Bekanntmachung des Hinweises auf die Patenterteilung: **04.06.2003**
(21) Anmeldenummer: 95110631.9
(22) Anmeldetag: 07.07.1995
(51) Int. Cl.: C12N 15/12, C07K 14/705, C12N 5/10, C07K 16/18, C12N 15/11, C12Q 1/00, G01N 33/50

(54) **Transportprotein, das den Transport von kationischen Xenobiotika und/oder Pharmaka bewirkt, dafür kodierende DNA-Sequenzen und deren Verwendung**
Protein for the transport of cationic xenobiotic and/or pharmaceutical compounds, DNA sequences coding therefore and its uses
Protéine de transport de substances pharmaceutiques et/ou xénobiotiques cationiques, ADN codant pour celle-ci et ses utilisations

(30) Priorität: 13.07.1994 DE 4424577
(43) Veröffentlichungstag der Anmeldung: 06.03.1996
(73) Patentinhaber: Aventis Pharma Deutschland GmbH, 65929 Frankfurt am Main (DE)
(72) Erfinder: Koepsell, Hermann, Prof Dr., D-97072 Würzburg (DE); Gründeman, Dirk, Dr., D-69123 Heidelberg (DE); Gorboulev, Valentin, Dr., D-97078 Würzburg (DE)

(56) Entgegenhaltungen:
- WO-A-93/08261
- GRUNDEMANN D ET AL: "Drug excretion mediated by a new prototype of polyspecific *transporter*." NATURE, DEC 8 1994, 372 (6506) P549-52, ENGLAND, XP002077625

## Beschreibung

Bei den Säugetieren und insbesondere beim Menschen werden kationische Pharmaka und Xenobiotika mit unterschiedlicher molekularer Struktur, Katecholamine und andere endogene Kationen durch polyspezifische Transportproteine in der Niere und Leber ausgeschieden, die in luminalen und basolateralen Plasmamembranen lokalisiert sind. Diese Transportproteine unterscheiden sich in funktioneller Hinsicht von den bereits bekannten Monoamintransportproteinen in neuronalen Plasmamembranen und synaptischen Vesikeln und von den ATP-abhängigen Exportproteinen für hydrophobe Pharmaka ("Multidrug"-Transportproteine).

Im Rahmen der vorliegenden Erfindung wurde zunächst eine komplementäre DNA-Sequenz aus Rattennieren isoliert, die für ein 556 Aminosäuren langes Membranprotein kodiert, das im folgenden als OCT1 bezeichnet wird. Dieses Transportprotein wirkt in der basolateralen Membran der proximalen Nierentubuli (Nierenkanälchen) und in Hepatozyten als Kationentransporter für verschiedene Zielmoleküle.

Das mit OCT1 bezeichnete Transportprotein ist nicht homolog zu irgendeinem anderen bisher bekannten Protein, weist eine bisher einzigartige Verteilung von hydrophoben und negativ geladenen Aminosäuren auf und wird ausschließlich in Nieren, Leber und Darm gefunden. Das OCT1-Transportprotein transportiert Kationen mit unterschiedlicher Struktur, wird durch eine Vielzahl von kationischen Substanzen mit unterschiedlicher Hydrophobizität gehemmt und besitzt andere funktionelle Eigenschaften als ein schon bekanntes polyspezifisches Transportprotein ("Multidrug-Transporter"), welches ausschließlich sehr hydrophobe Substanzen transportieren kann. Das Transportprotein OCT1 wird als ein neuer Prototyp eines polyspezifischen Transportproteins in Säugetieren angesehen.

Viele organische Kationen einschließlich häufig verwendeter Pharmaka wie Antihistaminika, Antiarrhythmika, Sedativa, Optiate, Diuretika, Zytostatika und Antibiotika werden in den Urin und in die Galle mittels aktiven Transports durch Nierenepithelzellen und Hepatozyten ausgeschieden. Bei der aktiven Sekretion in der Niere werden die Kationen durch polyspezifische Transportsysteme in der basolateralen und luminalen Plasmamembran der proximalen Nierentubuli transportiert. Beide Systeme unterscheiden sich in funktioneller Hinsicht. Die Transportproteine in der basolateralen Membran, die strukturell verschiedene Kationen wie Tetraethylammonium (TEA), N¹-Methylnicotinamid (NMN) und N-Methyl-4-phenylpyridinium (MPP) transportieren können, werden durch eine Vielzahl von strukturell unterschiedlichen extrazellulären Kationen inhibiert. Diese Transportproteine können durch ein im Inneren negatives Membranpotential und durch einen Gegentransport von intrazellulären Substraten angetrieben werden. In der luminalen Membran wurden zwei Transportsysteme beschrieben, die durch einen nach außer gerichteten Protonengradienten angetrieben werden, aber durch das Membranpotential nicht beeinflußt werden. Das eine dieser Transportsysteme hat eine breite Substratspezifität, die mit der des Kationentransportsystems in der basolateralen Membran von proximalen Nierentubuli vergleichbar ist. Aufgrund funktioneller Ähnlichkeiten wird angenommen, daß dieses polyspezifische Transportsystem der luminalen Membran mit dem extraneuronalen Transportsystem für Noradrenalin im Herzen identisch ist.

Gegenstand der vorliegenden Erfindung sind daher Transportproteine, die für den Transport von kationischen Xenobiotika und/oder Pharmaka aus dem Blut in die Leber- oder Nierenepithelzellen oder für den Transport von kationischen Xenobiotika oder Pharmaka aus dem Darm in den Blutkreislauf zuständig sind.

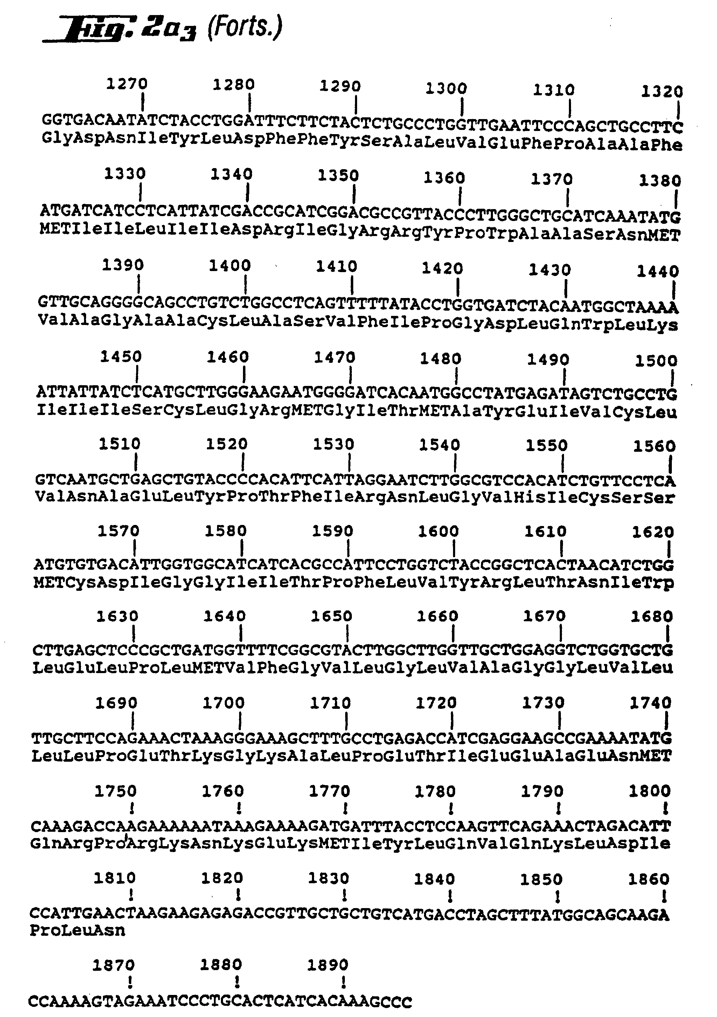

Die erfindungsgemäßen Transportproteine sind dadurch gekennzeichnet, daß sie die Aminosäuresequenz aufweisen, die in Figur 2a₁, Figur 2a₂ oder Figur 2a₃ dargestellt ist.

Gegenstand der vorliegenden Erfindung sind auch DNA-Sequenzen, die für ein erfindungsgemäßes Transportprotein kodieren. Die erfindungsgemäßen DNA-Sequenzen weisen sequenz die in Figuren 2a₁, 2a₂ oder 2a₃ gezeigten Nukleotid Sequenzen auf.

Von besonderer Bedeutung sind die erfindungsgemäßen Transportproteine und DNA-Sequenzen in der medizinischen und pharmakologischen Forschung. Mit Hilfe der erfindungsgemäßen DNA-Sequenzen ist es beispielsweise möglich, epitheliale Zellinien herzustellen, die ein erfindungsgemäßes Transportprotein permanent exprimieren. Hierzu wird mit an sich bekannten gentechnologischen Methoden die für das Transportprotein kodierende DNA-Sequenz in einen geeigneten Vektor eingebaut, mit dem eine geeignete epitheliale Zellinie, die das Transportprotein bisher nicht exprimierte, transformiert wird. Dadurch können Zellinien erhalten werden, die ein erfindungsgemäßes Transportprotein konstant exprimieren.

Derartige epitheliale Zellinien, die die Transportproteine exprimieren, können zur Testung der zu erwartenden renalen und biliären Ausscheidung sowie der intestinalen Resorption von kationischen Pharmaka und/oder Xenobiotika in vitro eingesetzt werden. Mit Hilfe derartiger Zellinien kann also bereits in vitro, d.h. ohne aufwendige Tierexperimente, festgestellt werden, ob und wenn ja in welchem Ausmaß Pharmaka oder auch andere biologisch aktive Wirkstoffe ausgeschieden oder aus dem Darm in den Blutkreislauf resorbiert werden.

Mit Hilfe der erfindungsgemäßen DNA-Sequenzen können solche Transportproteine isoliert werden, die zu den erfindungsgemäßen Transportproteinen homolog sind. Es ist daher möglich, die entsprechenden Transportproteine aus allen Säugerspezies und aus dem Menschen zu isolieren, die zu den erfindungsgemäß offenbarten Transportproteinen homolog sind. Zwei entsprechende humane Sequenzen wurden bereits ermittelt. Eine Möglichkeit, wie eine derartige Isolierung erfolgen kann, ist die inzwischen allseits bekannte Polymerase Kettenreaktion. Es müssen daher nur geeignete DNA-Sequenzen aus der in Fig. 2a₁, 2a₂ oder 2a₃ gezeigten Sequenz ausgewählt werden, die als Primer für die Polymerase Kettenreaktion dienen können. Mit Hilfe dieser Primer kann die Isolierung von homologen Transportproteinen ohne Schwierigkeiten erfolgen.

Eine weitere Verwendungsmöglichkeit der erfindungsgemäßen Transportproteine und/oder der erfindungsgemäßen epithelialen Zellinien ist die Entwicklung von kationischen Signalmolekülen, die an biologisch aktive Verbindungen, wie Pharmaka, angehängt werden können, um deren renale und biliäre Ausscheidung oder ihre intestinale Resorption zu verändern. Es ist hierdurch möglich, verschiedene chemische Strukturen dahingehend zu überprüfen, ob sie eine Ausscheidung des damit verbundenen Moleküls über die Niere bzw. Leber begünstigen und eine Resorption aus dem Darm In den Blutkreislauf fördern, ober ob sie das jeweilige Gegenteil bewirken.

Insbesondere können die erfindungsgemäßen Transportproteine auch zur Entwicklung von Antikörpern, insbesondere monoklonalen Antikörper, dienen, mit deren Hilfe die Aufnahme von Pharmaka in Nierentubuluszellen blockiert werden kann, um die Nephrotoxizität von kationischen Pharmaka zu erniedrigen.

Weiterhin kann aufgrund der erfindungsgemäßen Offenbarung die Entwicklung von spezifischen Pharmaka erfolgen, die die Ausscheidung von bestimmten anderen kationischen Pharmaka und/oder Xenobiotika beeinflussen. Hierdurch ist es möglich, pharmakologisch wirksame Substanzen zu entwickeln, die die Aufnahme von anderen Wirkstoffen beeinflussen. Eine derartige Beeinflussung kann entweder darin bestehen, daß die Aufnahme eines Wirkstoffes aus dem Darm gefördert oder verhindert wird oder daß die Ausscheidung eines Wirkstoffes in der Niere und Leber gefördert oder verhindert wird.

Eine weitere bevorzugte Verwendung der erfindungsgemäßen DNA-Sequenzen ist die Entwicklung von Antisense-Nucleotidsequenzen. Hierbei können Nucleotidsequenzen entwickelt werden, die die Transkription und/oder Translation der entsprechenden Gene dadurch verhindern, daß sie an die entsprechenden natürlich vorkommenden komplementären Nucleotidsequenzen binden.

Eine weitere bevorzugte Verwendung der erfindungsgemäßen DNA-Sequenzen ist ihre Verwendung in molekularen Testkits zur Diagnose von molekularen genomischen Defekten in renalen und/oder biliären Kationenausscheidungsmechanismen. In solchen molekularen Testkits können die DNA-Sequenzen in einer besonders bevorzugten Ausführungsform zur Durchführung der Polymerase Kettenreaktion verwendet werden. Dabei werden anhand der bekannten DNA-Sequenz Primersequenzen ausgesucht und synthetisiert, mit deren Hilfe in der Polymerase-Kettenreaktion das für den Kationentransporter des jeweiligen Patienten kodierende Gen amplifiziert und bezüglich genetischer Mutationen untersucht werden kann.

Anhand der nachfolgenden Beispiele, die jedoch die Erfindung nicht einschränken sollen, wird das Wesen der vorliegenden Erfindung näher erläutert.

### Beispiel 1

Für die Klonierung der für das Transportprotein kodierenden Gene wurden zunächst doppelsträngige cDNA mit glatten Enden von Poly(A)⁺ RNA aus Rattennieren hergestellt, wobei ein Notl-Oligo(dT)Primer für die Synthese des ersten Stranges verwendet wurde. Nachdem EcoRI-Adaptoren, die einen SP6 RNA Polymerase Promotor enthalten, an die cDNA angebracht wurden, wurde mit Notl verdaut, der Größe nach aufgetrennt (1,5 bis 2,3 kb) und in die EcoRI Restriktionsschnittstelle des Vektors pBluescript (Stratagene) eingebaut und anschließend in den E.coli-Stamm DH10B elektroporiert. Aus Transformantenpools wurde die Plasmid-DNA isoliert, mit Notl linearisiert und mit Hilfe der SP6-RNA Polymerase transkribiert. Die cRNA wurde durch Poly(A)⁺ Selektion gereinigt und in einer Konzentration von 20 bis 40 ng pro Oozyte injiziert. Die Oozyten wurden inkubiert und die durch NMN inhibierbare ¹⁴C-TEA-Aufnahme wurde gemessen. Durch ein gezieltes Suchverfahren wurde aus der Genbibliothek ein einzelner Klon isoliert, der das für einen Kationentransporter aus der Niere kodierende Gen enthielt. Die Isolierung dieses Klons war nur nach Optimierung und teilweiser Veränderung der angewandten Methoden möglich. Zum Sequenzieren identifizierter DNA wurden überlappende Restriktionsfragmente von OCT1 subkloniert und an beiden Strängen vollständig sequenziert.

Das aus einer Ratten-Nieren-Genbank isolierte Gen OCT1, welches aus einem 1.882 Basenpaar langen cDNA-Fragment bestand, wurde in Xenopus laevis Oozyten exprimiert. Hierbei wurden die Oozyten nach RNA-Injektion drei Tage mit 5 mM Hepes-Tris-Puffer, pH 7,5, 110 mM NaCl, 3 mM KCI, 2 mM CaCl₂, 1 mM MgCl₂ (im folgenden als ORi bezeichnet) inkubiert. Der Transport wurde durch Inkubation der Oozyten mit ¹⁴C-TEA (Tetraethylammonium) gemessen, das in ORi (22°C) gelöst wurde. Weiterhin wurden Versuche mit unterschiedlichen Na⁺ und K⁺ Konzentrationen sowie Versuche in der Gegenwart von Ba⁺⁺, bei verschiedenen pH-Werten und in Gegenwart unterschiedlicher Inhibitoren durchgeführt. Da bei den verwendeten ¹⁴C-TEA-Konzentrationen die durch exprimiertes OCT1-Protein verursachte Aufnahme in ORi-Puffer für mehr als 90 Minuten linear war, wurden die Aufnahmeraten nach 90-minütiger Inkubation bestimmt. Für die Messungen mit veränderten Konzentrationen an Na⁺, K⁺, H⁺ und in Gegenwart von Inhibitoren wurden die Oozyten zuerst 30 Minuten unter den entsprechenden Pufferbedingungen inkubiert und die Aufnahmeraten wurden dann während einer 30-minütigen Inkubationsperiode mit ¹⁴C-TEA bestimmt. Nach der Inkubation mit ¹⁴C-TEA wurde die Aufnahme unterbrochen und die Oozyten gewaschen und hinsichtlich der aufgenommenen Radioaktivität untersucht.

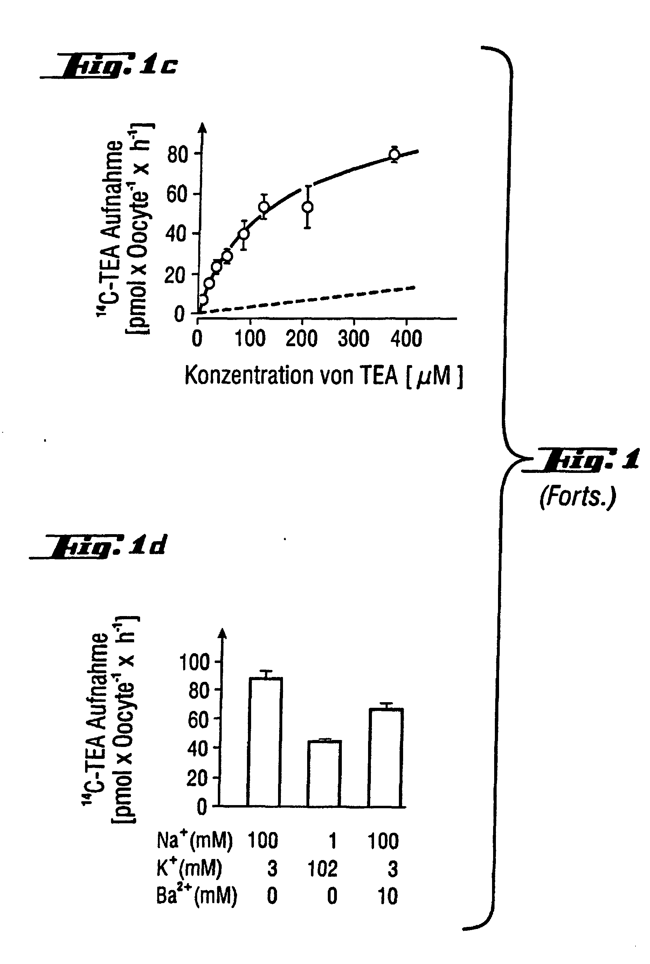

Es wurde also das 1.882 Basenpaar lange cDNA-Fragment unter Verwendung von Xenopus laevis Oozyten (wie oben beschrieben) exprimiert; das so exprimierte OCT1-Protein induzierte eine ¹⁴C-Tetraethylammonium (¹⁴C-TEA) Aufnahme, die durch NMN (N¹-Methylnicotinamid) inhibiert werden konnte, wobei die Aufnahme mehr als 250-fach über den Kontrollwerten lag, bei denen Wasser in die Oozyten injiziert wurde. Die Ergebnisse sind in Figur 1a graphisch dargestellt.

Die klonierte OCT1 cDNA enthält einen offenen Leserahmen, der für ein Membranprotein mit 556 Aminosäuren kodiert. Die Aminosäuresequenz ist in der Figur 2a₁ dargestellt. Sie zeigt keine Ähnlichkeiten mit den Proteinen in Datenbanken.

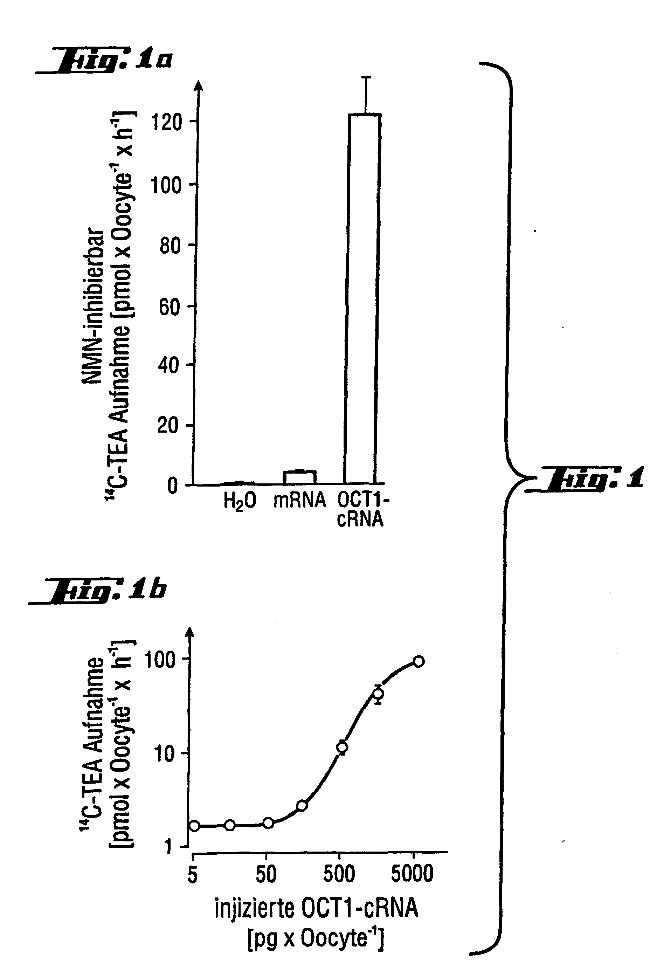

Die Expression der ¹⁴C-TEA-Aufnahme war abhängig von der Menge der injizierten OCT1-cRNA. Diese Ergebnisse sind in der Figur 1b dargestellt. Die cRNA-Abhängigkeit der exprimierten Aufnahme konnte durch die Hill-Gleichung mit n = etwa 2 beschrieben werden.

Die Substratabhängigkeit der von dem Transportprotein OCT1 bewirkten ¹⁴C-TEA-Aufnahme folgte der Michaelis Menten-Gleichung. Diese Ergebnisse sind in der Figur 1c dargestellt. Der geschätzte Kₘ-Wert von 95 ± µM ähnelte dem Kₘ-Wert (164 µM), der in früheren Versuchen für den Kationentransport über die basolaterale Membran proximaler Nierentubuli von Ratten bestimmt worden war. Er war 14 mal geringer als der scheinbare Kₘ-Wert für den polyspezifischen H⁺-Kationen-Gegentransporter in der Bürstensaummembran proximaler Nierentubuli von Ratten.

### Beispiel 2

Um weiterhin festzustellen, ob es sich bei dem OCT1-Transportprotein um das potentialabhängige polyspezifische Kationentransportsystem aus der basolateralen Membran oder um das potentialunabhängige polyspezifische H⁺-Kationen-Gegentransportsystem der Bürstensaummembran handelt, wurde untersucht, ob die von dem Transportprotein OCT1 bewirkte Aufnahme von dem Membranpotential bzw. von einem Protonengradienten über die Membran abhängig ist. Außerdem wurde die Hemmung der exprimierten ¹⁴C-TEA-Aufnahme durch verschiedene Inhibitoren untersucht.

Die Figuren 1d und 1e zeigen, daß die durch das Transportprotein OCT1 vermittelte ¹⁴C-TEA-Aufnahme vom Membranpotential abhängig ist, aber nicht wesentlich geändert wird, wenn ein nach innen oder außen gerichteter Protonengradient von einer pH-Einheit angelegt wird. Das OCT1-Transportprotein hat also die gleichen Grundcharakteristiken wie der über die basolaterale Membran der proximalen Nierentubuli gemessene Kationentransport.

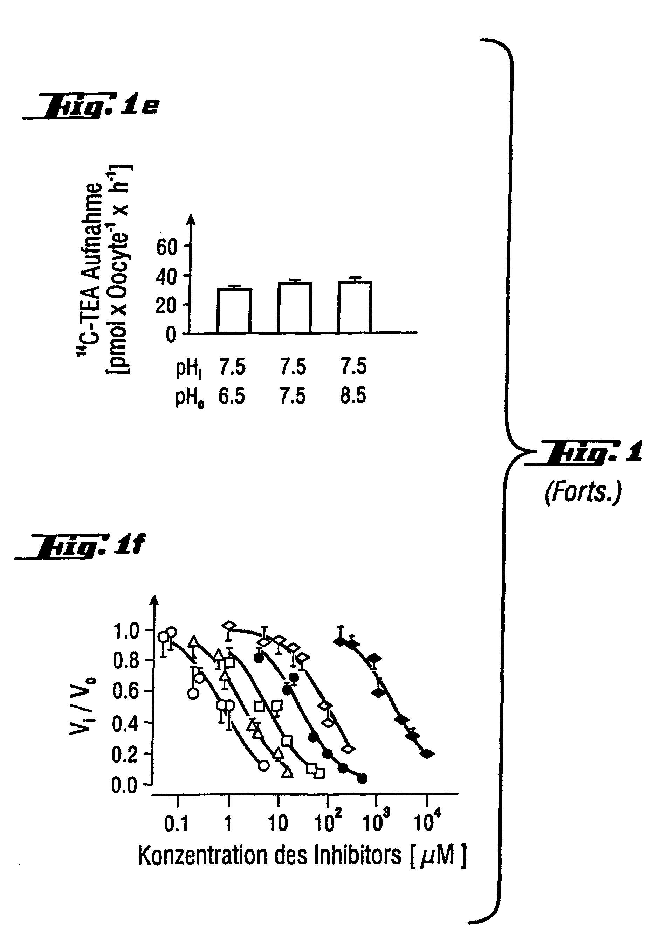

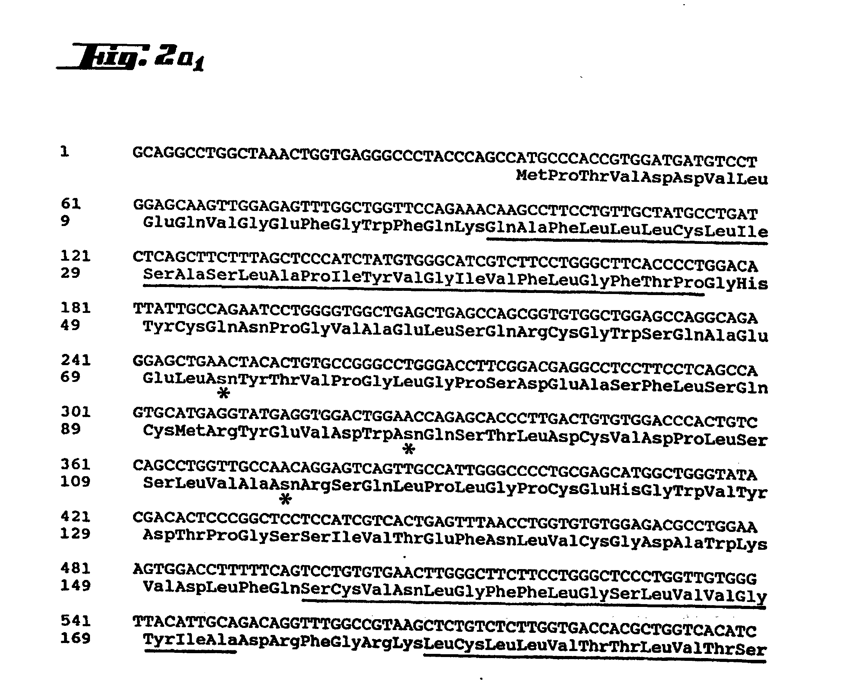

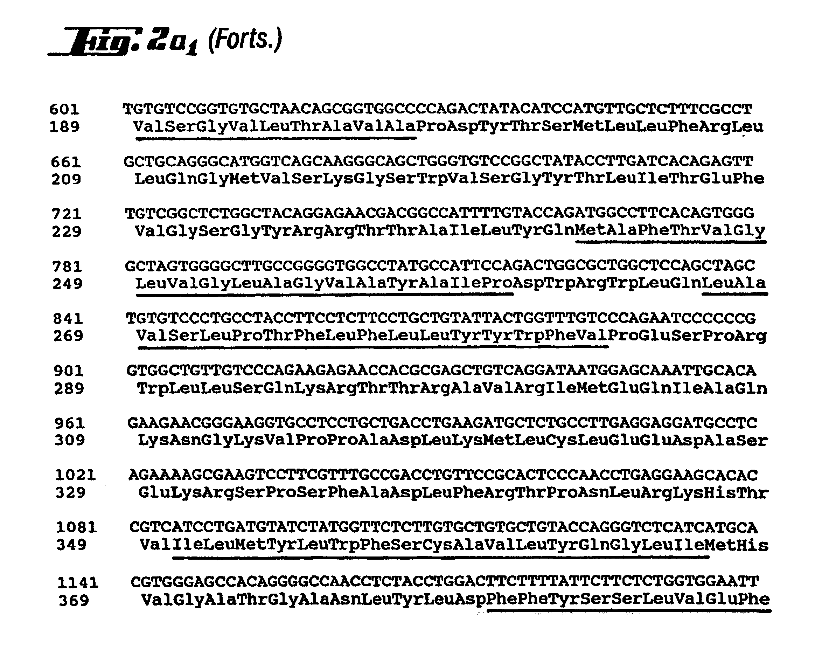

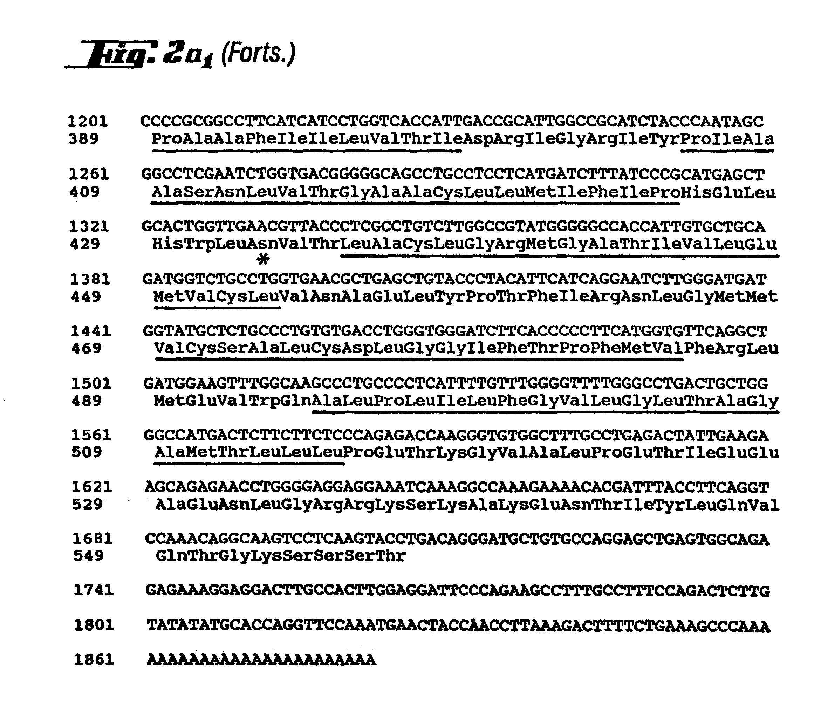

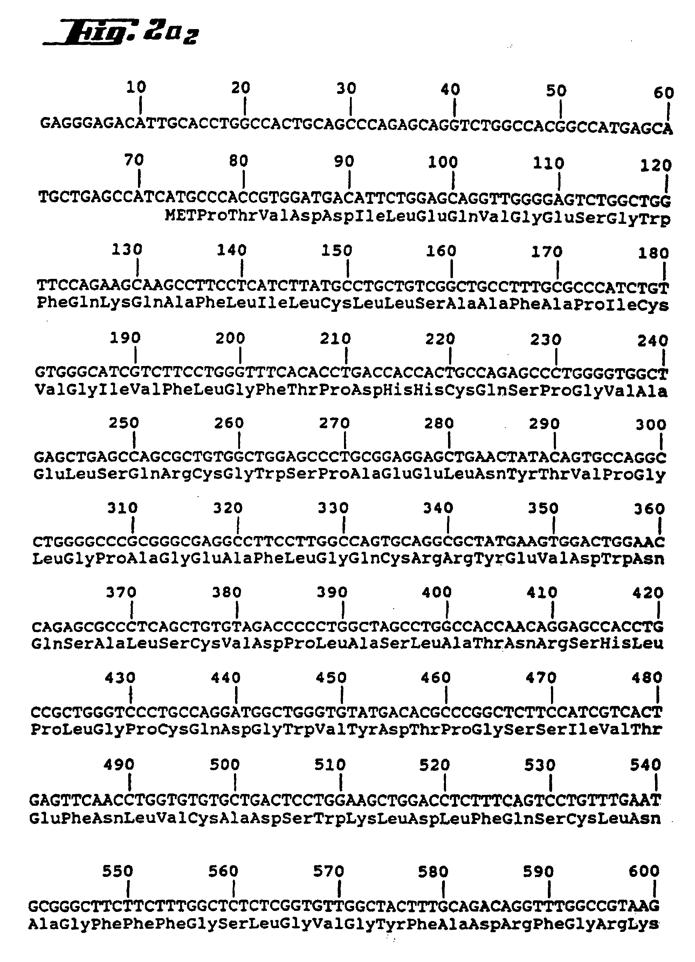

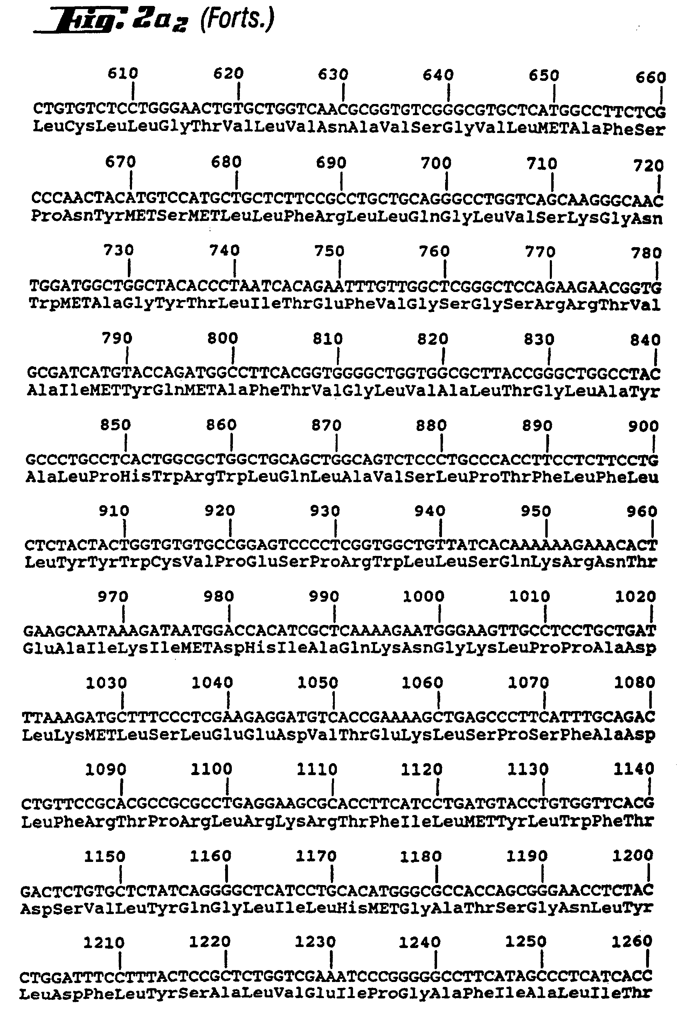

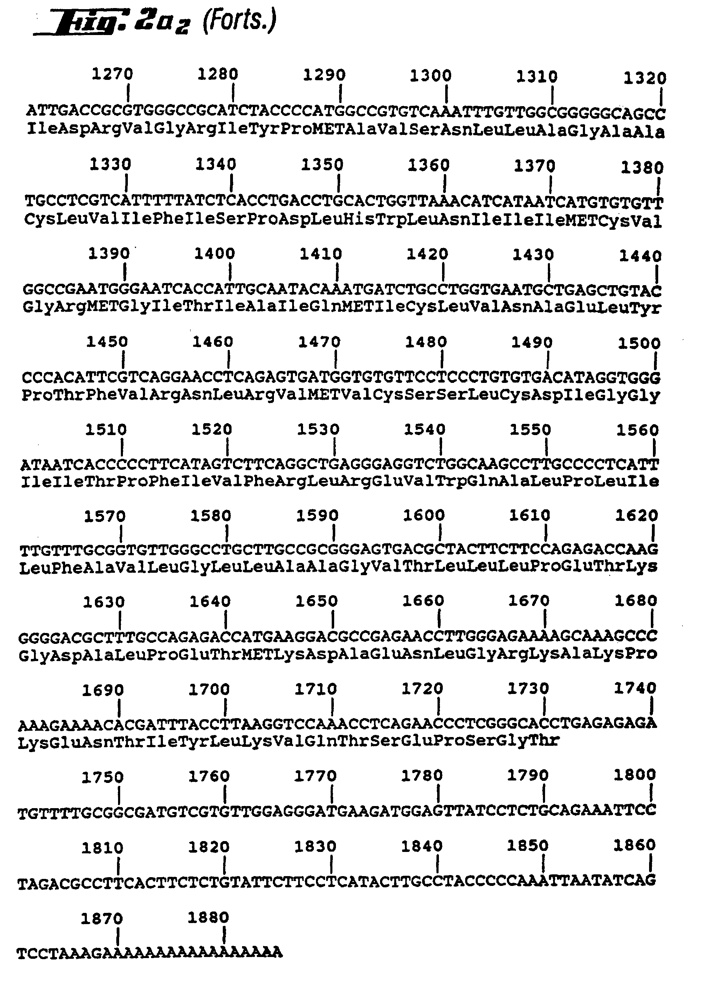

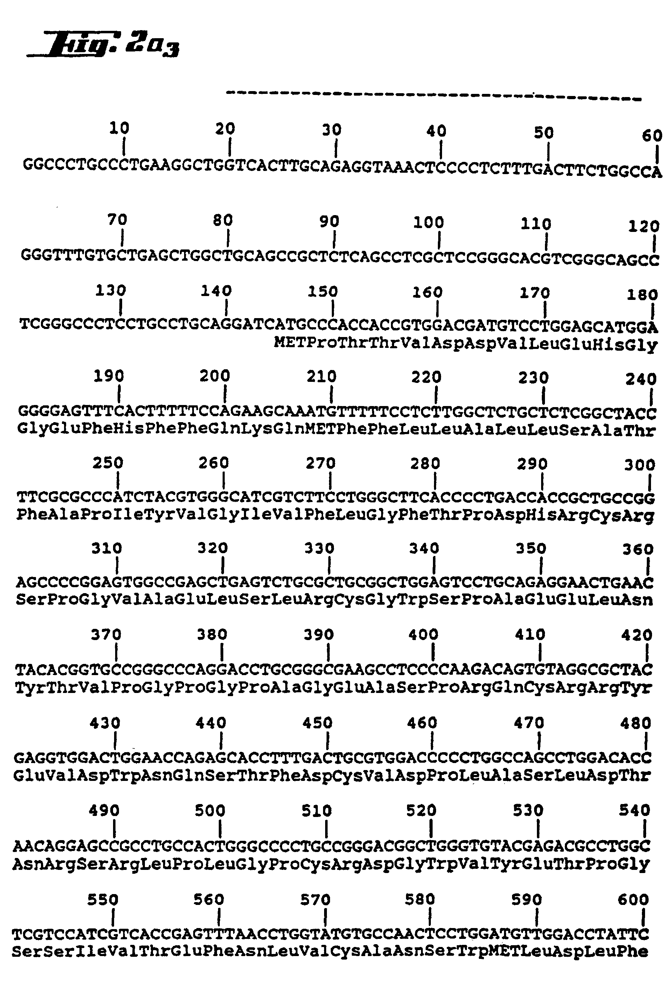

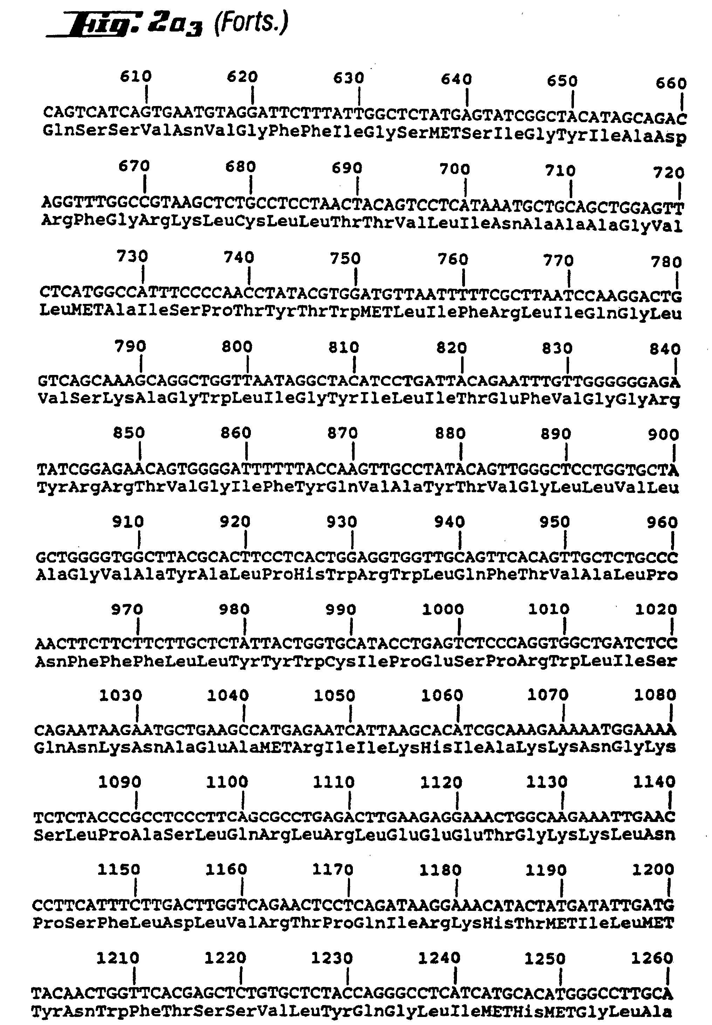

Figur 1f und Tabelle 1 zeigen, daß die durch OCT1 bewirkte ¹⁴C-TEA-Aufnahme durch organische Kationen mit verschiedener molekularer Struktur inhibiert wird. Diese Strukturen schließen mehrere häufig verwendete Pharmaka wie Chinin, Desipramin, Procainamid und O-Methyl-isoprenalin ein. Die geschätzten Kᵢ-Werte liegen zwischen 0,13 µM für 1-Ethyl-2([1,4-dimethyl-2-phenyl-6-pyrimidinyliden]methyl)chinoliniumchlorid (Cyanin 863) und 1 mM für Tetramethylammonium (TMA).

**Tabelle 1**

| | |
|---|---|
| Inhibitor | Kᵢ (µM) |
| Cyanin 863 | 0,13 ± 0,02 |
| Decynium 22 | 0,36 ± 0,08 |
| Tetrapentylammonium | 0,43 ± 0,09 |
| Chinin | 0,93 ± 0,08 |
| Desipramin | 2,8 ± 0,6 |
| Mepiperphenidol | 5,2 ± 0,3 |
| Procainamid | 13 ± 2 |
| 1-Methyl-4-phenylpyridinium | 13 ± 2 |
| Corticosteron | > 10 |
| Reserpin | > 20 |
| O-Methyl-isoprenalin | 43 ± 5 |
| Tetramethylammonium | 1000 ± 100 |
| N¹-Methylnicotinamid | 1000 ± 200 |

Tabelle 1 zeigt die Empfindlichkeit der ¹⁴C-TEA-Aufnahme in Xenopus laevis Oozyten, denen die cRNA des Nierentransportproteins OCT1 injiziert wurde.

Bei der Durchführung der Inhibitionsexperimente wurden den Xenopus laevis Oozyten 5 ng OCT1-cRNA injiziert und die Auswirkungen der in Tabelle 1 aufgeführten Inhibitoren bei 5 bis 8 unterschiedlichen Inhibitor-Konzentrationen auf die Aufnahme von 95 µM in die Oozyten gemessen. Die Werte wurden auch in Figur 1f dargestellt. Die Inhibierungskurven wurden durch nichtlineare Regressionsanalyse angepaßt und die Kᵢ-Werte (± SD) wurden bestimmt.

Im Unterschied zu dem schon bekannten polyspezifischen Transportprotein, dem sogenannten Multidrug-Transporter, welches ausschließlich durch hydrophobe Substanzen inhibiert wird, wurde das erfindungsgemäße Transportprotein OCT1 auch durch hydrophile Verbindungen wie TMA und NMN gehemmt. Desipramin inhibierte den durch OCT1 bewirkten Transport mit einem 700-fach höheren Kᵢ-Wert als den neuronalen Noradrenalin-Transport in Plasmamembranen von Nervenzellen. 5 µM Reserpin beeinflußt den durch OCT1 bewirkten Transport nicht, während das neuronale Monoamin-Transportprotein in synaptischen Vesikeln durch subnanomolare Reserpin-Konzentrationen inhibiert wird.

### Beispiel 3

Durch Vergleich der Kᵢ-Werte von OCT1 mit früher erhaltenen funktionellen Daten von Membranvesikeln und von Messungen mit kultivierten Nierenepithelzellen konnte die Identität des OCT1-Transportproteins mit dem basolateralen kationischen Transportprotein bestätigt werden. Bei solch einem Vergleich müssen die Spezies-abhängigen Unterschiede bei dem Kationentransport und die methodischen Beschränkungen der unterschiedlichen Verfahren zur Messung der Inhibierung des Kationentransports berücksichtigt werden. In früheren Untersuchungen wurde der Kationentransport in Rattennieren durch Mikroperfusionsexperimente bestimmt, die mit kurzen Inkubationszeiten (4 Sekunden) durchgeführt werden müssen. Da mit dieser Methode keine Diffusions-unabhängige Kᵢ-Bestimmung von Inhibitoren mit hoher Affinität möglich ist, haben wir uns auf den Vergleich niederaffiner Hemmstoffe beschränkt. Bei einem Vergleich der niederaffinen Inhibitoren TMA und NMN fanden wir, daß die Kᵢ-Werte des OCT1-exprimierten Transportproteins (etwa 1 mM) den Kᵢ-werten (TMA 1,4 mM, NMN 0,54 mM), die für die basolaterale TEA-Aufnahme in den proximalen Nierentubuli von Ratten gemessen wurden, entsprechen. Sie unterschieden sich deutlich von den Kᵢ-Werten (TMA 70 mM, NMN 8,3 mM), die für die luminale TEA-Aufnahme bestimmt wurden.

### Beispiel 4

Die basolaterale Lokalisierung von OCT1 wird zusätzlich durch den Kᵢ-Wert (0,4 µM) gestützt, der für die Inhibierung der von OCT1 bewirkten Aufnahme durch 1,1'-Diethyl-2,2'-cyaninjodid (Decynium 22) erhalten wurde. In LLC-PK1-Zellen wurde für den TEA-Transport über die luminale Membran ein K₁-Wert von 5,6 nM bestimmt, während der Kᵢ-Wert für den TEA-Transport über die basolaterale Membran mit > 0,1 µM geschätzt wurde. Um das OCT1-Transportprotein weiter zu charakterisierten, wurde getestet, ob MPP, das eine etwa 10-fach höhere Affinität hat als TEA, ebenfalls durch OCT1 transportiert wird. Nach Injektion von 8 ng OCT1-cRNA in Oozyten wurde eine spezifische ³H-MPP-Aufnahme exprimiert, die durch Chinin inhibierbar war. In einem Ansatz von Oozyten wurden ähnliche Vₘₐₓ-Werte für die exprimierte Aufnahme von ¹⁴C-TEA (148 ± 4 pmol x Oozyten⁻¹ x h⁻¹) und ³H-MPP (97 ± 5 pmol x Oozyten⁻¹ x h⁻¹) bestimmt. In Leberzellen ist die Existenz von polyspezifischen Kationentransportern beschrieben worden. Kürzlich wurde die MPP-Aufnahme in kultivierte Heptozyten gemessen. Dabei zeigte sich, daß etwa 90 % der MPP-Aufnahme durch die gleichen Hemmstoffe gehemmt werden konnten wie der durch OCT1 exprimierte Kationentransport. Die an den Hepatozyten für die MPP-Aufnahme bestimmten Kᵢ-Werte (O-Methylisoprenalin 78 µM, MPP 13 µM, Chinin 0,8 µM, Decynium 22 0,23 µM und Cyanin 863 0,10 µM) waren nahezu identisch mit den Werten, die für die TEA-Aufnahme durch das von Xenopus Oozyten exprimierte OCT1-Protein erhalten wurden. Diese Daten lassen vermuten, daß das OCT1-Transportprotein oder ein hochhomologes Transportprotein in der Plasmamembran von Hepatozyten vorhanden ist.

### Beispiel 5

Die Nucleotidsequenz und die Aminosäuresequenz von OCT1 ist in Figur 2a₁ dargestellt. Vor dem offenen Leserahmen sind Stoppkodons zu finden und eine Initiationsstelle der Translation von Kozak-Typ (ACGCCATG).

Eine Analyse der Hydrophilie/Hydrophobie von OCT1 ließ 11 hydrophobe α-helikale Bereiche erkennen, die die Membran voraussichtlich durchqueren. Eine Darstellung der Hydrophobie/Hydrophilie-lndices findet sich in Figur 2b. Die voraussichtlich membranspannenden Bereiche sind 17 bis 27 Aminosäuren lang. Sie sind durch einen langen, zwei mittellange und sieben kurze hydrophile Bereiche miteinander verbunden. Da drei potentielle N-Glycosilierungsstellen an dem hydrophilen Bereich zwischen den beiden ersten die Membran durchquerenden Proteinbereichen vorausgesagt wurden, wurde die in Figur 2c dargestellt Orientierung von OCT1 vorgeschlagen. Der erste hydrophile Bereich enthält 14 negativ geladene Aminosäuren, die für die Kationenbindung an OCT1 wichtig sein können.

### Beispiel 6

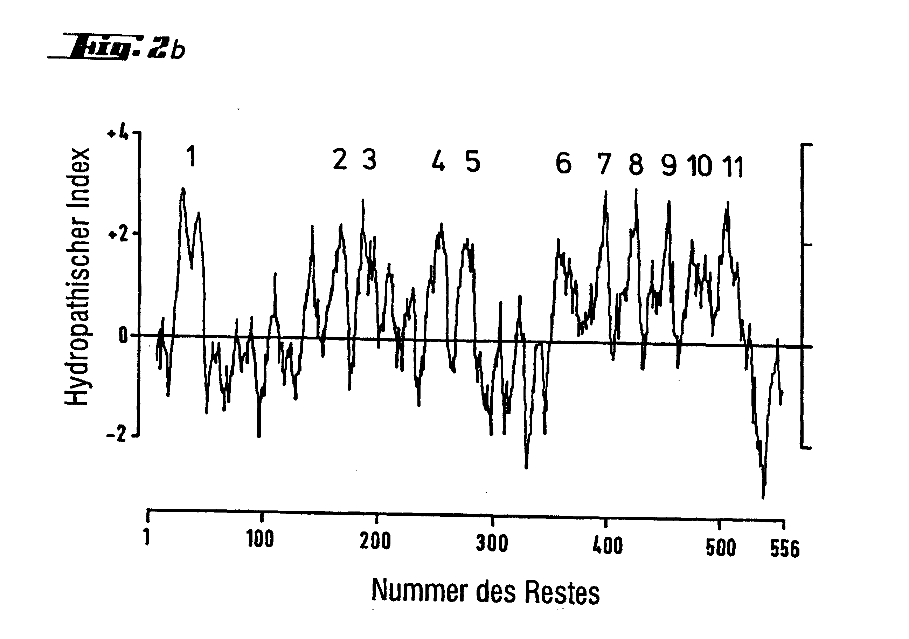

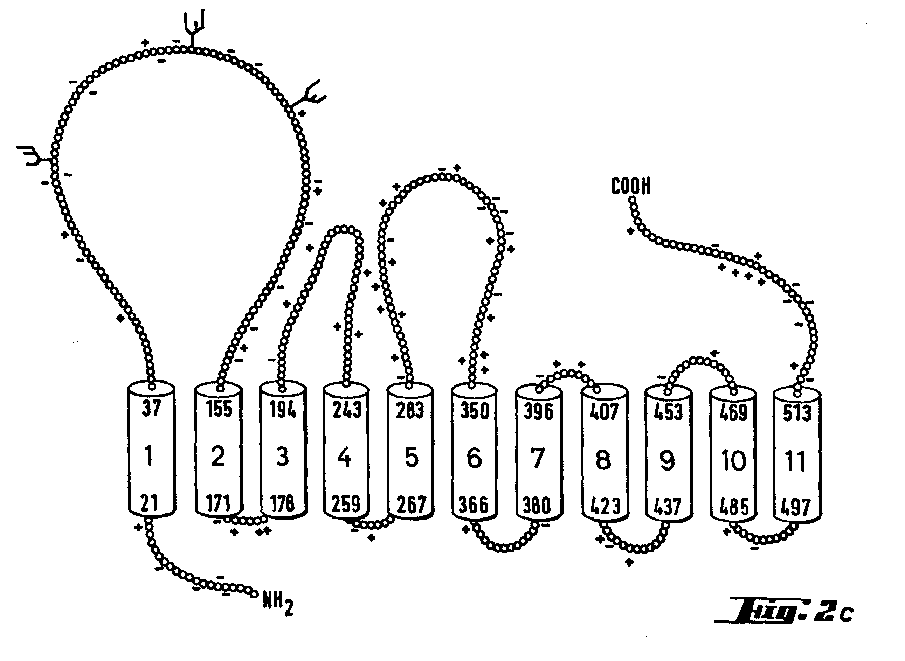

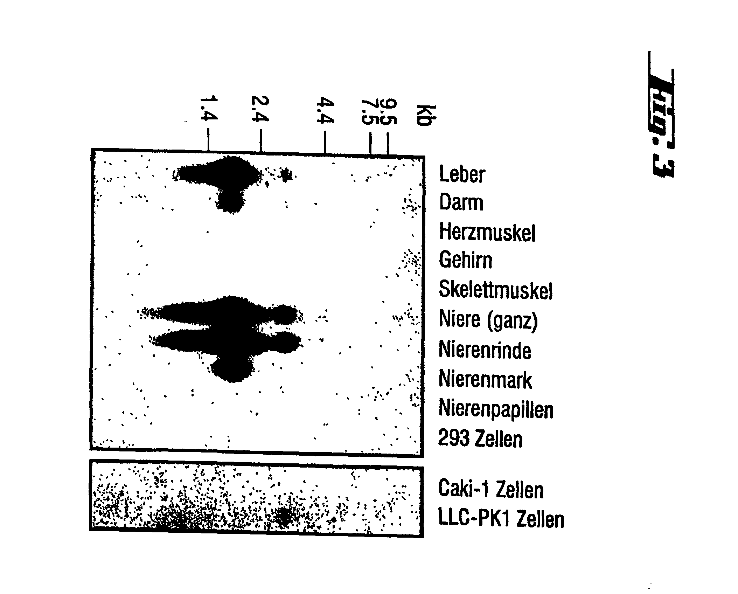

Verschiedene Rattengewebe und einige Zellinien wurden bezüglich der Lokalisation von für Transportprotein OCT1 spezifischer mRNA mit Hilfe des sogenannten Northern Blots analysiert. Hierzu wurde die gesamte RNA durch die Guanidinium-Phenol-Chloroform-Methode isoliert und die mRNA wurde unter Verwendung der Oligo(dT)-Zellulose-Chromatographie gereinigt. Die mRNA wurde mit Hilfe von Formaldehyd-Agarosegel-Elektrophorese fraktioniert, auf eine Hybond-N-Membran (Amersham) übertragen und anschließend hybridisiert. Hierzu wurden von den Rattenzellen und aus der Zellinie 293 5 µg und aus den Zellinien Caki-1 und LLC-PKI1 1,5 µg mRNA auf das Formaldehyd-Agarosegel aufgetragen. Die Hybridisierung erfolgte mit einem ³²P-markierten cDNA-Fragment der erfindungsgemäßen DNA-Sequenz von dem Plasmid pOCT1 (es wurden die Nucleotide 285 bis 1196 verwendet. Die Hybridisierung wurde für 18 Stunden bei 42°C in der Hybridisierungslösung durchgeführt (50 % Formamid, 5 x SSPE, 5 x Denhardt's Lösung, 0,5 % SDS und 20 µg Lachssperma DNA). Die Membran wurde in mehreren Schritten zu einer endgültigen Stringenz von 0,25 x SSPE, 0,1 % SDS bei 60°C gewaschen. Zur Darstellung der Zellinie LLC-PK1 erfolge eine Exponierung des Films für 24 Stunden und für die anderen Spuren erfolgte eine Exponierung des Films für sechs Stunden. Zur Ermittlung der Größe der RNA-Fragmente wurde ein RNA-Standard (0,14 bis 9,5 Kilobasen-Bereich von GIBCO/BRL) verwendet. Die Größen sind in der Figur 3 angegeben.

In Figur 3 ist die durch die Northern Blot Analyse erhaltene Autoradiographie dargestellt. Bei der Nierenrinde, dem Nierenmark, der Leber und dem Darm wurden deutliche Banden bei 1,9 Kilobasen und weitere Banden bei 3,4 und 4,8 Kilobasen beobachtet. In der Zellinie LLC-PK1 konnte nur eine Hybridisierung in dem Bereich von 3,4 Kilobasen beobachtet werden. Keine Signale für OCT1 konnten dagegen in der Nieren-Papille, im Skelettmuskel, im Herzmuskel, im Gehirn, in der menschlichen embryonalen Nierenzellinie 293 und In Caki-1-Zellen beobachtet werden. Da Herz- und Caki-1-Zellen das extraneuronale Noradrenalin-Transportprotein enthalten, welches wahrscheinlich mit dem H ⁺ -Kationen-Gegentransportprotein auf luminalen Nierenmembranen identisch ist, gehören die Kationen-Transportproteine in der basolaterialen und luminalen Membran der proximalen Nierentubuli wahrscheinlich zu verschiedenen genetischen Familien. In situ-Hybridisierungen zeigten, daß das OCT1-Transportprotein in den proximalen Nierentubuli, in den Epithelzellen der Leber und in den Enterozyten des Dünndarms exprimiert werden.

Die obigen Beispiele zeigen, daß ein neues und einzigartiges Protein kloniert wurde, das eine bedeutende Role bei der Eliminierung von kationischen Pharmaka in Niere und Leber spielt. Vermutlich ist dieses Protein auch an der Reabsorption von kationischen Verbindungen im Darm beteiligt. Obwohl der Kationentransport und die Exkretion von Pharmaka seit mehr als 30 Jahren intensiv untersucht werden, konnten in der Vergangenheit nur geringe Fortschritte erzielt werden. Der Grund dafür ist, daß die Ausscheidung von Pharmaka in Leber und Nieren den Transport über die basolaterale und die luminale Plasmamembran der epithelialen Zellen einschließt und daß diese Transportprozesse durch funktionell verschiedene Kationen-Transportproteine bewirkt werden. Darüber hinaus kann nicht ausgeschlossen werden, daß sowohl in der luminalen als auch in der basolateralen Nierenmembran verschiedene Kationen-Transportproteine mit ähnlicher Substratspezifität existieren. Durch die Klonierung des erfindungsgemäßen OCT1-Transportproteins wurde ein Typ des Kationen-Transportproteins identifiziert. Damit wurden viele Möglichkeiten zur weiteren Erforschung der Ausscheidung kationischer Pharmaka eröffnet.

### Beispiel 7

Mit Hilfe der in der vorliegenden Anmeldung beschriebenen Techniken konnten zwei zu OCT1 homologe menschliche Gene kloniert und vollständig bzw. teilweise sequenziert werden. Das vollständig sequenzierte Gen (HOCT1) besteht aus 1885 Basen und kodiert für ein Protein mit 553 Aminosäuren. Es ist in Figur 2a₂ dargestellt. Zwischen den Aminosäuren von OCT1 und HOCT1 besteht 78 % Identität. Das andere menschliche Gen (HOCT2) besteht aus 1896 Basen und kodiert für ein Protein mit 555 Aminosäuren. Die Nucleotidsequenz und die abgeleitete Aminosäuresequenz von OCT2 ist in Figur 2a₃ dargestellt. Zwischen den Aminosäuren von OCT1 und HOCT2 besteht eine Identität von 68 %.

### Erläuterung der Zeichnungen

Figur 1 zeigt die Expression von OCT1 in Xenopus laevis Oozyten. Die angegebenen ¹⁴C-TEA-Aufnahmeraten stellen die Mittelwerte von 10 bis 20 Messungen ± Standardabweichung dar.
Figur 1a zeigt einen Vergleich der NMN-inhibierten ¹⁴C-TEA-Aufnahme, die nach Injektion von Wasser, 20 ng Rattennieren mRNA oder 10 ng cRNA von OCT1 beobachtet wurde. Die Konzentrationen an ¹⁴C-TEA und NMN in den Inkubationsmedien betrugen 200 µM bzw. 10 mM.
Figur 1b zeigt die Aufnahmeraten von 200 µM ¹⁴C-TEA nach Injektion von verschiedenen Mengen an cRNA von OCT1. Die Kurve wurde durch Anpassung der Hill-Gleichung an die erhaltenen Daten errechnet (n = 1,9 ± 0,2).
Figur 1c zeigt die Substratabhängigkeit der ¹⁴C-TEA-Aufnahme, die nach Injektion von 3 ng OCT1-cRNA pro Oozyte exprimiert wurde. Die durchgehende Linie zeigt die Gesamtaufnahme, die eine sättigbare Komponente und eine lineare Komponente enthält, die in mit Wasser injizierten Kontrolloozyten bestimmt wurde. Die lineare Komponente wurde mittels linearer Regression angepaßt (gestrichelte Linie, 30 fmol x h⁻¹ x Oozyte⁻¹ × µM⁻¹). Die sättigbare Komponente wurde durch die Michaelis Menten Gleichung angepaßt (Kₘ 95 ± 10 µM, Vₘₐₓ 81 ± 5 pmol x h⁻¹ x Oocyte⁻¹). Die ausgezogene Linie wurde durch Anpassung an eine Gleichung, welche beide Komponenten enthält, errechnet.
Figur 1d zeigt die Potentialabhängigkeit der ¹⁴C-TEA-Aufnahme in Oozyten, denen 3 ng OCT1-cRNA injiziert wurden. Die Aufnahme von 95 µM ¹⁴C-TEA wurde in Gegenwart der angegebenen Konzentrationen von Na⁺, K⁺ und Ba²⁺ gemessen. Unter diesen Bedingungen lagen die Membranpotentiale zwischen -40 und -60 mV (100 mM Na⁺, 3 mM K⁺), 0 bis -10 mV (1 mM Na⁺, 102 mM K⁺) und zwischen -18 und -22 mV (100 mM Na⁺, 3 mM K⁺, 10 mM Ba²⁺).
Figur 1e zeigt die Aufnahme von 95 µM ¹⁴C-TEA in Anwesenheit und Abwesenheit von Protonengradienten bei Oozyten, denen 3 ng OCT1-cRNA injiziert wurden. Um durch Protonengradienten verursachte Änderungen des Membranpotentials zu verhindern, welche die ¹⁴C-TEA-Aufnahme verändern würden, wurden die Messungen in Gegenwart von 102 mM K⁺ und 1 mM Na⁺ im Inkubationsmedium durchgeführt. Dadurch wurde das Membranpotential auf etwa 0 mV gebracht. pH-Messungen mit Mikroelektroden zeigten, daß sich während der 30-minütigen Aufnahmeperiode der pH-Wert um weniger als 0,1 Einheiten änderte.
Figur 1f zeigt die Inhibierung der durch OCT1 bewirkten ¹⁴C-TEA-Aufnahme durch Decynium 22 (o), Chinin (Δ), Desipramin (□), Procainiamid (●), O-Methylisoprenalin (◇) und Tetramethylammonium (◆). Den Oozyten wurden 5 ng OCT1-cRNA injiziert und die Messungen wurden mit 95 µM ¹⁴C-TEA durchgeführt.
Figur 2a₁ zeigt die Nucleotidsequenz und die davon abgeleitete Aminosäuresequenz von OCT1. Diejenigen Bereiche, die vermutlich Transmembranbereiche sind, wurden unterstrichen und potentielle N-Glycosilierungsstellen vom Typ NXT/S werden durch Sternchen angegeben.
Figur 2a₂ zeigt die Nucleotid- und Aminosäuresequenz eines homologen Human-Genes aus der Niere (HOCT1). Das dargestellte Genstück umfaßt 1885 Basen und kodiert für 553 Aminosäuren.
Figur 2a₃ zeigt eine Nucleotid- und Aminosäuresequenz eines zweiten homologen Human-Genes aus der Niere (HOCT2). Das dargestellte Genstück umfaßt 1856 Basen und kodiert für 555 Aminosäuren.
Figur 2b zeigt eine Kyte-Doolittle Hydrophobie/Hydrophilie-Analyse von OCT1 unter Verwendung eines Fensters von 9 Aminosäuren. Die Bereiche, die vermutlich Transmembranbereiche sind, wurden mit 1 bis 11 numeriert.
Figur 2c stellt eine schematische Darstellung von OCT1 dar. Die Aminosäurereste Arg, Lys und His werden durch Pluszeichen angegeben und die Aminosäurereste Glu und Asp durch Minuszeichen gekennzeichnet. Potentielle Glycosilierungsstellen in der ersten hydrophilen Schleife sind kenntlich gemacht.
Figur 3 zeigt die Lokalisierung von für OCT1 spezifischer mRNA in verschiedenen Rattengeweben und in einigen Zellinien.

## Patentansprüche

1. Transportprotein, das für den Transport von kationischen Xenobiotika und/oder Pharmaka aus dem Blut in die Leber- oder Nierenepithelzellen oder für den Transport von kationischen Xenobiotika und/oder Pharmaka aus dem Darm zuständig ist, **dadurch gekennzeichnet, daß** es die in Figur 2a₁, 2a₂ oder 2a₃ dargestellten Aminosäuresequenzen aufweist.

2. Isolierte DNA-Sequenz, **dadurch gekennzeichnet, daß** sie für ein Transportprotein nach Anspruch 1 kodiert.

3. Verwendung einer DNA-Sequenz nach Anspruch 2 zur Herstellung einer epithelialen Zellinie, die ein Transportprotein nach Anspruch 1 konstant exprimiert.

4. Epitheliale Zellinie, die ein Transportprotein nach Anspruch 1 exprimiert.

5. Epitheliale Zellinie nach Anspruch 4 zur Testung der zu erwartenden renalen und biliären Ausscheidung sowie der intestinalen Resorption von kationischen Pharmaka und/oder Xenobiotika in vitro.

6. Verwendung einer DNA-Sequenz nach Anspruch 2 zur Isolierung von zu Transportproteinen gemäß Anspruch 1 homologen Transportproteinen.

7. Verwendung nach Anspruch 6, **dadurch gekennzeichnet, daß** die Isolierung mit Hilfe der Polymerase Kettenreaktion erfolgt.

8. Verwendung der Transportproteine nach Anspruch 1 und/oder der epithelialen Zellinie nach Anspruch 4 oder 5 zur Entwicklung von kationischen Signalmolekülen, die an biologisch aktive Verbindungen, wie Pharmaka, angehängt werden können, um deren renale und biliäre Ausscheidung oder ihre intestinale Resorption zu verändern.

9. Verwendung des Transportproteins nach Anspruch 1 und/oder der epithelialen Zellinie nach Anspruch 4 oder 5 zur Entwicklung von Antikörpern, mit deren Hilfe die Aufnahme von Pharmaka in Nierentubuluszellen blockiert werden kann, um die Nephrotoxizität von kationischen Pharmaka und/oder Xenobiotika zu erniedrigen.

10. Verwendung des Transportproteins nach Anspruch 1 und/oder der epithelialen Zellinie nach Anspruch 4 oder 5 zur Entwicklung von spezifischen Pharmaka, mit deren Hilfe die Aufnahme von anderen Pharmaka und/oder Xenobiotika in Nierentubuluszellen blockiert werden kann, um die Nephrotoxizität von kationischen Pharmaka zu erniedrigen.

11. Verwendung einer DNA-Sequenz nach Anspruch 2 zur Entwicklung einer Antisense-Nucleotidsequenz, mit deren Hilfe die Aufnahme von Pharmaka und/oder Xenobiotika in Nierentubuluszellen blockiert werden kann, um die Nephrotoxizität von kationischen Pharmaka zu erniedrigen.

12. Verwendung einer DNA-Sequenz nach Anspruch 2 in molekularen Testkits zur Diagnose von molekularen genetischen Defekten in renalen und biliären Kationenausscheidungsmechanismen.

13. Verwendung nach Anspruch 12, **dadurch gekennzeichnet, daß** das molekulare Testkit diejenigen Komponenten enthält, die zur Durchführung der Polymerase Kettenreaktion benötigt werden.

## Claims

1. A transport protein which is responsible for transporting cationic xenobiotics and/or pharmaceuticals from the blood into the liver epithelial cells or kidney epithelial cells or for transporting cationic xenobiotics and/or pharmaceuticals from the intestine, which transport protein exhibits the amino acid sequences depicted in Figure 2a₁, 2a₂ or 2a₃.

2. An isolated DNA sequence, which encodes a transport protein as claimed in claim 1.

3. The use of a DNA sequence as claimed in claim 2 for preparing an epithelial cell line which constantly expresses a transport protein as claimed in claim 1.

4. An epithelial cell line which expresses a transport protein as claimed in claim 1.

5. An epithelial cell line as claimed in claim 4 for the in-vitro testing of the renal and biliary excretion and the intestinal resorption of cationic pharmaceuticals and/or xenobiotics which are to be expected.

6. The use of a DNA sequence as claimed in claim 2 for isolating transport proteins which are homologous with transport proteins as claimed in claim 1.

7. The use as claimed in claim 6, wherein the isolation is effected using the polymerase chain reaction.

8. The use of the transport proteins as claimed in claim 1 and/or of the epithelial cell line as claimed in claim 4 or 5 for developing cationic signal molecules which can be attached to biologically active compounds, such as pharmaceuticals, in order to alter their renal and biliary excretion or their intestinal absorption.

9. The use of the transport protein as claimed in claim 1 and/or of the epithelial cell line as claimed in claim 4 or 5 for developing antibodies which can be used to block the uptake of pharmaceuticals into renal tubule cells in order to decrease the nephrotoxicity of cationic pharmaceuticals and/or xenobiotics.

10. The use of the transport protein as claimed in claim 1 and/or of the epithelial cell line as claimed in claim 4 or 5 for developing specific pharmaceuticals which can be used to block the uptake of other pharmaceuticals and/or xenobiotics into renal tubule cells in order to decrease the nephrotoxicity of cationic pharmaceuticals.

11. The use of a DNA sequence as claimed in claim 2 for developing an antisense nucleotide sequence which can be used to block the uptake of pharmaceuticals and/or xenobiotics into renal tubule cells in order to decrease the nephrotoxicity of cationic pharmaceuticals.

12. The use of a DNA sequence as claimed in claim 2 in molecular test kits for diagnosing molecular defects at the genetic level in renal and biliary cation excretion mechanisms.

13. The use as claimed in claim 12, wherein the molecular test kit contains the components which are necessary for carrying out the polymerase chain reaction.

## Revendications

1. Protéine de transport, qui est responsable du transport de xénobiotiques et/ou de produits pharmaceutiques cationiques, du sang dans les cellules épithéliales du foie ou des reins, ou encore du transport de xénobiotiques et/ou de produits pharmaceutiques cationiques à partir de l'intestin, **caractérisée en ce qu'**elle présente les séquences d'acides aminés présentées sur les Figures 2a₁, 2a₂ ou 2a₃.

2. Séquence d'ADN isolée, **caractérisée en ce qu'**elle code pour une protéine de transport selon la revendication 1.

3. Utilisation d'une séquence d'ADN selon la revendication 2 pour fabriquer une lignée de cellules épithéliales qui exprime d'une manière constante une protéine de transport selon la revendication 1.

4. Lignée de cellules épithéliales qui exprime une protéine de transport selon la revendication 1.

5. Lignée de cellules épithéliales selon la revendication 4, destinée à tester l'excrétion rénale et biliaire à laquelle on peut s'attendre, ainsi que la résorption intestinale de composés pharmaceutiques et/ou de xénobiotiques cationiques in vitro.

6. Utilisation d'une séquence d'ADN selon la revendication 2 pour isoler des protéines de transport homologues des protéines de transport selon la revendication 1.

7. Utilisation selon la revendication 6, **caractérisée en ce que** l'isolement s'effectue à l'aide d'une réaction en chaîne par polymérase.

8. Utilisation des protéines de transport selon la revendication 1 et/ou de la lignée de cellules épithéliales selon la revendication 4 ou 5 pour mettre au point des molécules signal cationiques, qui peuvent être suspendues à des composés biologiquement actifs, tels que des composés pharmaceutiques, pour modifier leur excrétion rénale et biliaire ou leur résorption intestinale.

9. Utilisation de la protéine de transport selon la revendication 1 et/ou de la lignée de cellules épithéliales selon la revendication 4 ou 5 pour mettre au point des anticorps à l'aide desquels il est possible de bloquer l'absorption de produits pharmaceutiques par des cellules des tubules rénaux, pour diminuer la néphrotoxicité de produits pharmaceutiques et/ou de xénobiotiques cationiques.

10. Utilisation de la protéine de transport selon la revendication 1 et/ou de la lignée de cellules épithéliales selon la revendication 4 ou 5 pour mettre au point des produits pharmaceutiques spécifiques à l'aide desquels il est possible de bloquer l'absorption d'autres produits pharmaceutiques et/ou xénobiotiques par des cellules des tubules rénaux, pour diminuer la néphrotoxicité de produits pharmaceutiques cationiques.

11. Utilisation d'une séquence d'ADN selon la revendication 2 pour mettre au point une séquence nucléotidique antisens, à l'aide de laquelle il est possible de bloquer l'absorption de produits pharmaceutiques et/ou de xénobiotiques par des cellules des tubules rénaux, pour diminuer la néphrotoxicité de produits pharmaceutiques cationiques.

12. Utilisation d'une séquence d'ADN selon la revendication 2 dans des trousses d'essai moléculaire, pour le diagnostic de défauts génétiques moléculaires dans des mécanismes rénaux et biliaires d'excrétion de cations.

13. Utilisation selon la revendication 12, **caractérisée en ce que** la trousse d'essai moléculaire contient les composants qui sont nécessaires à la mise en oeuvre de la réaction en chaîne par polymérase.
